# EUROPEAN PATENT APPLICATION

(11) **EP 4 736 826 A1**
(43) Date of publication of application: **06.05.2026**
(21) Application number: 24210870.2
(22) Date of filing: 05.11.2024
(51) Int. Cl.: A61F 13/49, A61F 13/505

(54) **GUSSET FOR WASHABLE ABSORBENT UNDERGARMENT**

(71) Applicant: Essity Hygiene and Health Aktiebolag, 405 03 Göteborg (SE)
(72) Inventor: KNÖS, Anna, 405 03 GÖTEBORG (SE); BLOMSTRÖM, Philip, 405 03 GÖTEBORG (SE)
(74) Representative: Nederlandsch Octrooibureau

(57) **Abstract**

A washable absorbent undergarment comprising woven or knitted fabrics, has a multi-layer gusset extending at least within a crotch region of the garment. The gusset comprises at least a body-facing top layer, a moisture barrier layer and a garment layer, with lateral seams joining side edges of the layers together. The seams are formed in an everted state and subsequently reversed such that the side edges terminate at an interior of the gusset.

## Description

### Field

The present disclosure relates to washable absorbent undergarments comprising an integrated absorbent assembly in the form of a gusset. Such articles are intended for reusable sanitary use in absorbing body fluids such as urine and vaginal fluids. The disclosure also relates to such a gusset and methods of manufacture thereof.

### Background

A washable absorbent undergarment may be worn for the purpose of absorbing body fluids such as urine and vaginal fluids. Such garments form an environmental and economical advantageous alternative to disposable sanitary napkins and disposable absorbent undergarments. A washable absorbent undergarment typically comprises fabric panels of woven or knitted materials. Conventionally such a washable absorbent undergarment will also comprise an integral absorbent assembly located in the crotch region of the wearer when the undergarment is worn, sometimes referred to as the gusset. After use the absorbent undergarment is washed or laundered before reuse. In general, it would be desirable that garments can be washed and reused at least 10 times without disintegration. An example of such a washable absorbent undergarment is found in WO2023169665A1.

Depending upon the intended use, the gusset may vary in size and construction. It may extend over a smaller or larger area of the garment, e.g. depending on whether it is intended for daytime or night-time use. It may also be thicker or thinner, with additional wicking, distribution, barrier and absorbing layers. Nevertheless, in addition to being functional from an absorbing perspective, the resulting undergarment must inspire confidence and be comfortable to wear for prolonged periods as well as providing a good fit and being aesthetically pleasing. It should also be reusable multiple times without visible deterioration in terms of colour, shape, elasticity or construction.

Joining the different layers of the gusset together and to the garment material has been one of the major challenges in developing such undergarments. Although such garments are reusable a number of times, in order to be competitive in replacing other solutions, the garments must be simple and cost effective to produce and adequately able to secure against leakage. In particular, stitching through layers may lead it to leak. Manufacturing procedures such as external seams and tapes used for conventional garments are available but not always convenient.

It would thus be desirable to improve the construction of such undergarments and their components.

### Summary

This object may be at least partially achieved with a washable absorbent undergarment according to claim 1, a multi-layer gusset according to claim 13 and a method of manufacturing a multi-layer gusset according to claim 14, and further specified as set out in the dependent claims and in the following description.

The disclosure thus concerns a washable absorbent undergarment comprising woven or knitted fabric, having a multi-layer gusset extending at least through a crotch region of the garment. The gusset comprises at least a body-facing top layer, a moisture barrier layer and a garment layer with lateral seams joining side edges of the layers together. The seams are formed in an everted state of the gusset, such that once the gusset is turned to its position of use, the side edges terminate at an interior of the gusset and the barrier layer will form an edge seal of the gusset. In the present context, the term gusset is used to refer to what may form a crotch panel of the garment, stretching between a user's legs from a front side of the garment to a rear side of the garment. In some circumstances, this may be referred to as an integrated absorbent assembly although this term may exclude the garment layer itself.

Furthermore, the term everted is distinguished from terms such as reversed or turned inside-out, which might refer to the undergarment being inside-out. Everted is used for the case of a tubular structure that is turned inside out by passing the complete structure through its own lumen or opening. The gusset, with only the side edges joined together, forms such a tubular structure. It should be pointed out that it forms a number of potential lumens or openings between each pair of adjacent layers. As a result of the proposed everting of the gusset, the seam and in particular the side edges extending beyond the seam, are located at an interior of the gusset and not exposed at an exterior of the garment. Everting takes place at a lumen corresponding to the top layer, such that the top layer forms the body facing surface of the undergarment.

As used herein, the term "absorbent undergarment" refers to garments that are worn and intended to be placed against the skin of the wearer with the intended function of absorbing and containing body fluids such as urine and vaginal fluids including menstrual fluid, without significant visible external trace. The undergarment may for example be underwear, underpants, a panty, or swimwear of fabric. The undergarment is intended for teenage and adult users. It will be understood that most conventional garments will have a limited degree of absorbency but that for conventional undergarments the containment of body fluids is not intended, and an unexpected leakage of any significant volume would be perceptible externally either as a damp patch or discoloration that may penetrate through an outer garment.

The washable absorbent undergarment according to the present disclosure is also thus washable, i.e. intended to be laundered or otherwise restored after use for repeated reuse as a sanitary article. By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g. to 30°C or 60°C. The term is also intended to mean that the absorbent undergarment can be washed and reused more than once. Washable may be understood as being suitable to undergo at least 10 washing cycles at a temperature of at least 30°C, without disintegrating. During the washing, the absorbent assembly releases the absorbed fluids, thereby regenerating the absorptive functioning, enabling the absorbent assembly to be reused for the same purpose again. A disposable absorbent article is not constructed to be washable and reusable and is thus for single use only. A disposable absorbent article is made up of materials such as nonwoven materials and laminates, plastic films, superabsorbent polymer particles and cellulose fluff fibres.

The undergarment may be of any suitable form and reference above and in the following to 'garment' is not intended to mean other than an undergarment i.e. an innermost garment intended for being in contact with the body of a user. The garment has a waist opening and two leg openings. It is however not excluded that closure elements e.g. between the waist opening and the leg openings may be provided, allowing easy removal or donning of the garment. The undergarment may have various designs, such as briefs, boxer, hipster, high waist, string, Brazilian, or other suitable and conventional undergarment designs.

A central longitudinal axis of the undergarment is defined along the undergarment from a rear region and towards a front region and a transversal crotch axis of the undergarment is defined in a direction extending between the leg openings. The transversal crotch axis may be located at the narrowest point of the garment and/or may be located at the mid-point of the central longitudinal axis

The seams may be of any appropriate construction, depending on the materials used for the respective layers and the manufacturing procedure. Seams may include stitching, riveting, welding, adhesion and combinations thereof. Additional materials may be present such as tape, in the understanding that once completed, the seam and any such additional materials are largely concealed at an interior of the gusset. Given that one primary objective of the disclosed construction is to simplify the structure, a preferred seam is the stitched seam. The stitches may be of any suitable type including running, back, chain, cross, blanket or overlock stitches. Overlock stitched seams may be provided, using 2, 3 of 4 needles to ensure a secure connection.

As a consequence of the manner in which the seam is produced, the garment layer, sometimes named body fabric, is sandwiched between the barrier layer and the top layer at the location of the seam. By everting the gusset through the lumen formed between the top layer and the garment layer, these two layers become the outermost layers, while the barrier layer is fully enclosed between them.

All of the layers of the gusset may have the same width dimension in the width direction of the gusset. In particular, they may have the same width dimension measured along the respective layer from seam to seam. Once everted to the position of use, a join between the outermost layers may then be positioned at a side edge of the gusset i.e. facing laterally outwards. The exact orientation of the join may depend on the relative extensibility of the respective layers. The barrier layer and garment layer may be provided to be wider than the top layer, at least in the crotch region. In this context, wider is intended to refer to the width dimension measured along the respective layer from seam to seam. It will be understood that excess material present beyond the seam is largely irrelevant to the subsequent orientation of the join. By providing a wider garment layer and barrier layer, these layers may wrap or extend around the side edges of the gusset. In particular, having the barrier layer envelop the gusset may assist in preventing leakage. This construction can also be used to ensure that joins between the garment layer and the top layer extend along a body facing surface of the garment at a distance from the side edge of the gusset. This may also provide for a neater and more comfortable fit.

The position of the joins can be adjusted as required by adapting the relative widths of the layers. The joins may be on the body facing surface at a distance of at least 2 mm from the side edges of the gusset or at least 5 mm from the side edges of the gusset. The garment layer and barrier layer may be at least 2 mm wider than the top layer. The position of the join with respect to the side edge may also vary in a longitudinal direction of the gusset.

The gusset may have any appropriate shape and size. It may be dimensioned such that at least a crotch region of the undergarment is covered during use. The crotch region is a surface section having a size and shape to cover some or all of the genital area of a user, i.e. a surface section shaped to fit in between the legs of a user, that has a length sufficient to extend at least from the mons pubis to the perineum and a width that varies along the length. The crotch region thus has a width substantially corresponding to a transversal width in between leg openings of the undergarment.

The area of the garment in which the gusset is present may be adapted according to the particular garment and the intended use. The gusset may cover an area of at least 10 cm² of the garment, such as between 10 cm² and 400 cm², or between 50 cm² and 100 cm².

The gusset may be of any appropriate shape according to the intended use and the shape of the undergarment. It may be dog-bone shaped, having curved sides and slightly enlarged ends. It may be four-sided, having a front edge, a rear edge and two side edges with the side edges coincident with respective leg openings. The gusset may be rectangular. In this context, "rectangular" is intended to denote that the assembly has an outer contour that is a four-sided shape, having two pairs of sides that are generally parallel to one another. The rectangular gusset or its component layers can be easily cut multiple times from a main material source (often a large sheet or roll of fabric or foil material), with a single cutting line forming two edges of adjacent components. Thus, the use of rectangular shaped gussets reduces the amount of cutting and minimizes waste. Furthermore, the cutting of rectangular contours from a main material source allows for multiple components to be cut directly adjacent to one another, without having to account for a positioning direction of each individual component in the manufacturing line. Thus, no energy is wasted during production to account for variation in direction of each component.

At least 75% of the total length of the edges of the at least one rectangular shaped gusset may be straight edges. Thus at least 75% of the outer contour of the rectangular shaped gusset conforms to the rectangular shape, thereby limiting the amount of material wasted, while allowing for some minimal shape variations in the layers that are required to assure wearer comfort and prevent the article from bulging during use.

The gusset may have a waisted shape, being narrowest at the crotch region and wider at a front edge and a rear edge. Such a shape is better adapted for integration with the remainder of the undergarment. The minimum width at the crotch region should be adequate to allow everting of the gusset during manufacture. In further alternatives, the gusset may be trapezoidal or even triangular extending only in a front crotch region to a minimum width at a transversal crotch axis that may be 30 mm or less. It will be understood that in this case everting can only take place in one sense and may be termed reversing. In this context, everting is intended to include the reversal of a triangular or conical shape.

In order to ensure an acceptable fit of the undergarment and in particular to reduce leakage, the undergarment may comprise leg elastics extending fully or partially around the leg openings. The leg elastics may extend along the lateral seams of the gusset. The leg elastics may be integrated into the seams of the gusset.

The gusset may be defined by the portion of the garment in which the above-mentioned at least three layers are present and joined together by the lateral seams. Individual layers may extend beyond the area defined by the gusset. In particular, the garment layer may extend beyond the gusset. The garment layer may comprise a single fabric panel extending throughout the undergarment. The fabric panel may comprise a front region, a rear region and a gusset region corresponding to the location of the gusset. The garment layer may be connected to itself at the hips to define leg openings and a waist opening.

The garment layer is washable and may be of any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the garment layer may be constructed of synthetic fibres or mixtures thereof selected from the group consisting of polyamide, acrylic, polyester. Further, the garment layer may be constructed of a blend or a mixture of naturally derived and/or synthetic fibres. The fibres may be recycled fibres. The garment layer may comprise a stretchable fabric, e.g. elastane, so that the absorbent undergarment can provide a firm fit while at the same time adapting to the wearer's movements thus preventing any leakage from migrating through the leg openings and keeping the gusset in place. The fabric of the undergarment comprises a woven or knitted fabric.

The garment layer may be connected to further fabric panels to form the garment. Any number of fabric panels may be present, including 1, 2, 3, 4, 5 or more fabric panels. One or more of these fabric panels may be present as the garment layer in the gusset. Connection may be achieved using conventional joining techniques, in particular by sewing. The further fabric panels may comprise a front panel and a rear panel, which may also be connected to each other at the hips to define leg openings and a waist opening. The further fabric panels may be of the same fabric as the garment layer or of different fabrics e.g. selected from the above-mentioned fabrics.

The moisture barrier layer is washable and functions to prevent moisture from penetrating through the gusset to the garment layer. The moisture barrier layer may be an extruded polymer layer. Suitable moisture impermeable materials may include thermoplastic films, such as polyurethane or the like. Alternatively, it may comprise a weave or knit. The moisture barrier layer may also comprise a moisture impermeable material laminated or coated onto a woven or knitted material. The moisture barrier layer may have a surface weight of between 50 and 300 gsm, such as between 75 gsm and 200 gsm. Although the moisture barrier layer may be impervious to liquid water, the moisture barrier layer may be breathable. It may have a breathability or moisture vapor transmission rate (WVTR) of more than 500g. WVTR is measured by the rate at which water vapor passes through, in grams of water vapour per square meter of fabric per 24-hour period (g/m²/d), often abbreviated to just "g".

The wearer facing top layer is that area of the gusset that is in contact with the body of the user. It may comprise a water-permeable material allowing the body fluids to migrate into the gusset. The top layer may be constructed of any suitable fabric, including natural and/or naturally derived fibres selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibres selected from the group consisting of polyamide, acrylic, polyester, or elastane, such as a mixture of polyester and elastane. Further, the top layer may be constructed of a blend or a mixture of naturally derived and/or synthetic fibres. The materials used for construction of the top layer should be soft and non-irritating to the skin and be readily penetrated by any body fluids. The top layer is washable and of a knitted or woven fabric. The top layer may comprise between 20% and 100% natural or naturally derived fibres, such as between 40% and 100% natural or naturally derived fibres and such as between 60% and 100% natural or naturally derived fibres. The top layer may have a basis weight of 80-300 gsm.

The top layer may be co-extensive with the gusset i.e. equal in the longitudinal direction to a length of the barrier layer. Alternatively, the top layer may extend beyond the moisture barrier layer at the front side and/or at the rear side of the garment. In this case, the lateral seams connecting the garment layer to the top layer may also extend beyond the moisture barrier layer.

At least one layer of the gusset may be absorbent to ensure the effective take up of bodily fluids. The absorbency may be integrated into the top layer or into the moisture barrier layer. There may be provided at least one discrete absorbent core layer. The absorbent core layer may be located between the top layer and the barrier layer in the gusset. In the initial everted state, the top layer may be located beneath absorbent core layer. In the seam, the absorbent core layer and the moisture barrier layer may together sandwich the top layer and the garment layer.

The core layer is washable and may comprise any suitable absorbent material including woven and knitted fabrics. The core layer may be e.g. a double-knit material or a terry fabric.

The core layer may further be defined by various properties or combinations of properties. The core material may have a basis weight of 120-600 g/m². This basis weight may be achieved by a single layer of core material although it is not excluded that multiple layers may be provided to increase the basis weight.

Various materials and yarns may be implemented in the core layer, including any material capable of absorbing fluid, such as microfibre or polymer fibres, hydrophilic fibres, absorbent fibres and the like. In the present context, naturally derived fibres include fibres that have been synthesised from bio-based materials such as cellulose. Such materials include e.g. regenerated cellulose, viscose, lyocell, rayon, Tencel and modal. Natural fibres further include cotton, linen, wool, silk, hemp, jute, bamboo, ramie, coir, banana and the like. Synthetic materials such as e.g. polyester may also be used.

The yarns forming the core layer may have any appropriate construction or weight to achieve the desired absorption capacity. These may be spun yarns of short staple fibres and may be single-ply or multi-ply. The yarns may be relatively fine to favour a close knit or weave that will not easily unravel.

The core layer may be co-extensive with the gusset. The core layer may be smaller than the gusset. The core layer may extend less at the front side and/or at the rear side than other layers of the gusset. It may also extend laterally to a lesser extent than the other layers and may not be joined to the other layers within the lateral seams. The core layer may be retained within the gusset by other means. It may be joined only to the top layer e.g. by adhesion, welding, stitching or the like. Alternatively, it may be adhered to the moisture barrier layer e.g. by adhesion, welding, stitching or the like. It may be retained only at its lateral edges or also at its front and rear edges or over all or part of its upper or lower surface. The core layer may also be loosely retained within the interior of the gusset e.g. merely by its shape. In particular, for a waisted gusset, the core layer may be held by its waist.

The gusset may comprise further layers. In particular, it may comprise a moisture distributing or wicking layer, which may be provided directly underneath the top layer. The wicking layer may have wicking features to allow the moisture to spread away from the wearer and into the core. Wicking enables an efficient spread of the moisture, allowing the moisture to be received into a wider area of the underlying core. This feature is particularly relevant for users suffering from stress incontinence as spurts of urine may cause the core to be saturated rapidly at the point of impact. The wicking layer may be constructed of any suitable fabric, including natural and/or naturally derived fibres or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer may be constructed of synthetic fibres or mixtures thereof selected from the group consisting of polyamide, acrylic, polyester or elastane. Further, the wicking layer may be constructed of a blend or a mixture of natural, naturally derived and/or synthetic fibres. According to the present disclosure, the wicking layer should be launderable and comprise woven or knitted fabrics. The wicking layer may comprise between 20% and 100% natural or naturally derived fibres, The basis weight of the wicking layer may be 180-250 gsm.

Other layers or partial layers may also be present, or the above layers may be further complemented with other functionalities. In particular, material in strip or tape form may be inserted into the seam between any of the previously mentioned layers. A strip of hydrophobic material, such as a hydrophobic fabric strip, may be inserted into the seam between the garment layer and the top layer. When everting the gusset an outer edge of the hydrophobic strip will be located over the top layer. This can help prevent moisture from the top layer from wicking to the garment layer. The outer edge of the fabric strip, not held within the seam, may be attached to the garment layer by stitching or tape/adhesive. This may take place at any point in the manufacturing process, avoiding damage to the moisture barrier layer.

The disclosure also relates to a multi-layer gusset, comprising woven or knitted fabrics, for a washable absorbent undergarment. The gusset may comprise at least a body-facing top layer, a moisture barrier layer and a garment layer, with lateral seams joining side edges of the layers together. The seams are formed in an everted state such that the side edges terminate at an interior of the gusset. The gusset may have a front edge and a rear edge that can be joined to a respective front garment panel and rear garment panel to form the undergarment. The gusset may be located at least at a crotch region of the undergarment. The gusset may be further as explained above or hereinafter.

The disclosure still further relates to method of manufacturing a washable multi-layer gusset comprising woven or knitted fabrics, for a washable absorbent undergarment. The method is a simple and cost-effective method of producing an absorbent undergarment. The method may comprise providing a stack of fabrics comprising in sequence, a moisture barrier layer, a garment layer, a body-facing top layer and an optional absorbent core layer. Subsequently, the side edges of the layers are joined together to form lateral seams. The seams may be stitched seams, such as overlock stitched seams. The stack is then everted by passing all of the layers through a lumen formed between the top layer and the garment layer. The resulting gusset has the top layer and garment layer outermost with the moisture barrier layer fully enclosed at an interior of the gusset.

The moisture barrier layer, garment layer, body-facing top layer and optional absorbent core layer may be provided from continuous supplies of material e.g. from a roll. The layers may be individually cut to size before forming the stack or the stack may be formed, and the edges of the stack may be subsequently trimmed before or after joining the side edges.

Joining of the side edges may take place in any appropriate manner, such by stitching, e.g. overlock stitching. Everting of the stack may then either take place by hand or be automated by machine.

Once the gusset is formed, manufacture of the undergarment may take place by attaching the garment layer to further fabric panels of garment material or to itself.

### Brief description of the drawings

A washable absorbent undergarment according to the present disclosure will be described by way of example, with reference to the attached drawings, in which:
Fig. 1 shows a front view of an exemplary absorbent undergarment comprising a multi-layer gusset;
Fig. 2 shows a top view of the undergarment of Fig. 1 in a flat laid-out state with the side joints between the rear region and the front region removed;
Fig. 3 is a cross-section through the absorbent undergarment of Fig. 2 along the line III-III;
Fig. 4 is a cross-section of the stack of gusset layers before everting;
Fig. 5 is a view corresponding to Figure 4 during everting of the gusset; and
Fig. 6 is a top view of an alternative undergarment.

### Description

Different aspects of the present disclosure will be described more fully hereinafter with reference to the enclosed drawings. The absorbent undergarment disclosed herein can, however be realized in many different forms, such as different sizes and absorption levels, and should not be construed as being limited to the aspects set forth herein. In all figures of the following detailed description, the same reference numerals will be used to indicate the same elements.

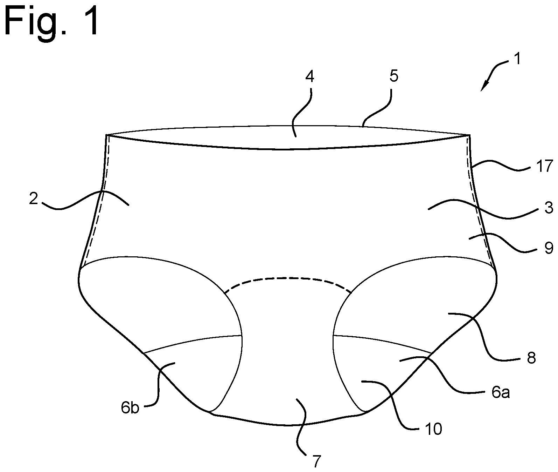

Fig. 1 illustrates as an example a washable and reusable absorbent undergarment 1. As outlined in the above, the undergarment 1 as proposed herein may however have various designs, such as briefs, boxer, hipster, high waist, string, Brazilian, or other suitable undergarment designs. The undergarment 1 may be designed for teenage or adult use.

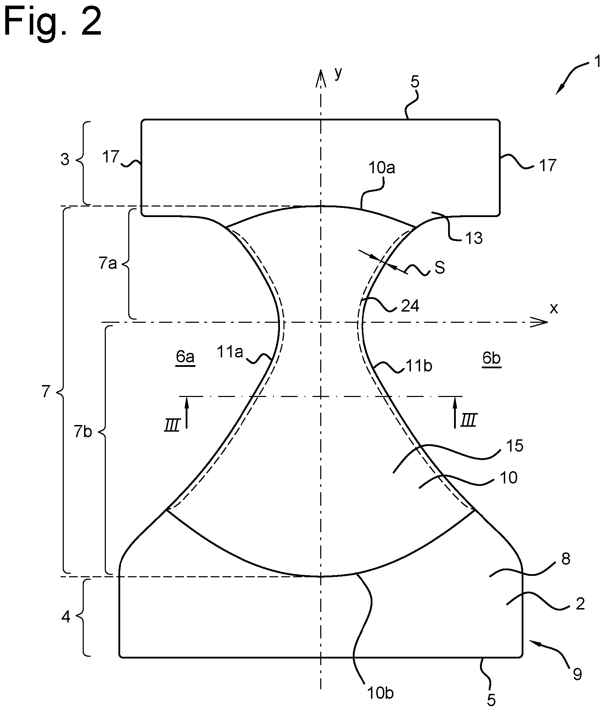

The undergarment 1 comprises a fabric panel 2 comprising woven or knitted fabrics having a front region 3, a back region 4 and a crotch region 7 extending between the front and back regions 3, 4. The front region 3 and the back region 4 are joined at the hips such that the undergarment 1 forms a waist opening 5 and a pair of leg openings 6a, 6b. As such, the front region 3 will be visible from the front of the user when the undergarment 1 is worn, and the back region 4 will be visible from the back of the user when the undergarment 1 is worn. The undergarment of the Fig. 1 and Fig. 2 example comprises one fabric panel 2, which has been cut to form the front region 3, the rear region 4 and the crotch region 7. However, in other variants, a plurality of fabric panels may be joined so as to form the front region 3, the rear region 4 and the crotch region 7. For example, the undergarment 1 may comprise a front panel, a rear panel and a crotch panel. The one or more fabric panels may all comprise the same fabric, giving the undergarment a unitary appearance, or the one or more panels may comprise different fabrics, for example to provide aesthetically pleasing undergarments comprising e.g. lace fabric.

The fabric of the fabric panel 2 may be any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric may be breathable to allow vapor to escape from the wearer's skin and from the absorbent structure.

In the illustrated example, the fabric is a single layer of interlock knitted fabric that terminates at the waist opening 5, without any waist band or additional elastic. A characteristic of interlock knitted fabric is that it can be cut, and the cut edge is stable against fraying without requiring any form of seam. It will nevertheless be understood that the absorbent undergarment 1 may comprise a waist band, arranged along the waist opening 5 of the absorbent undergarment 1, which may be elasticated and/or provided with a frictional inner surface.

To join the front region 3 and the back region 4 so as to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or conventional mechanical bonds, such as stitching techniques. As illustrated in Fig. 1, the fabric panel 2 has an outside surface 9 facing away from the wearer and an inside surface 8 facing in a direction towards the wearer. A multi-layer gusset 10 is located in the crotch region 7.

In Fig. 2, the absorbent undergarment 1 of Fig. 1 is shown in a flat laid-out state, where the side joints 17 of the undergarment 1 are removed. As seen in this laid-out state, a central longitudinal axis y is defined along the fabric panel 2 in a direction from the rear side 4 of the undergarment 1 towards the front side 3 of the undergarment 1. In Fig. 2, the absorbent undergarment 1 is shown from a wearer-facing side of the article 1. As illustrated in Fig. 1, the fabric panel 2 has an exterior side 9 facing away from the wearer and an interior side 8 facing in a direction towards the wearer. Fig. 2 thus displays the undergarment 1 in a flat laid-out state as seen from the interior side 8. Further, a transversal crotch axis x of the undergarment 1 is defined in a direction extending between the leg openings 6a, 6b, the transversal crotch axis x being perpendicular to the central longitudinal axis y at the crotch, being the position of minimum distance between the leg openings 6a, 6b.

The gusset 10 comprises a wearer facing top layer 15 that visibly illustrates to a user the extent of the gusset 10, with respect to a garment layer 13 provided by the fabric panel 2. A join 24 is visible between the garment layer 13 and the top layer 15 as will further be explained below. The transversal crotch axis x divides the crotch region 7 into a front crotch region 7a extending longitudinally forwards from the transversal crotch axis x to a front edge 10a of the gusset 10, and a rear crotch region 7b extending longitudinally between the transversal crotch axis x and a rear edge 10b of the gusset 10. The longitudinal extension of the gusset 10 may depend for example on the design of the undergarment 1.

The gusset 10 thus defines the crotch region 7, being generally at a location where the need for absorbance is most prevalent. For undergarments intended primarily for night-time use, the gusset 10 may extend further rearwards and more of the gusset 10 may be located to the rear of the transversal crotch axis x extending further upwards towards the waist opening 5.

As exemplified by the illustrated undergarment 1, the gusset 10 also forms a pair of leg edges 11a, 11b. Each leg edge 11a, 11b, thus connects the front edge 10a and the rear edge 10b. Each leg edge 11a, 11b defines the contour of the respective leg opening 6a, 6b of the undergarment 1. The joins 24 follow the leg edges 11a, 11b and are spaced by a distance S, which in the illustrated example is a constant value of 2 mm.

The skilled person will recognise that the shape of the gusset 10 may alternatively be adapted to various absorption needs and to various designs of the undergarment 1, so as to provide sufficient absorption and satisfactory fit. For example, the leg edges 11a, 11b may be straight or may comprise concave portions, as seen towards the central longitudinal axis y. Thus, the gusset 10 may be better adapted to fit between the wearer's legs. The front edge 10a of the gusset 10 may be curved convex as shown. This may be beneficial for the fit of the undergarment to the body and contribute to the avoidance of unwanted creases in the fabric panels. The front edge 10a may also be straight to reduce production and material costs. The same may apply to the rear edge 10b.

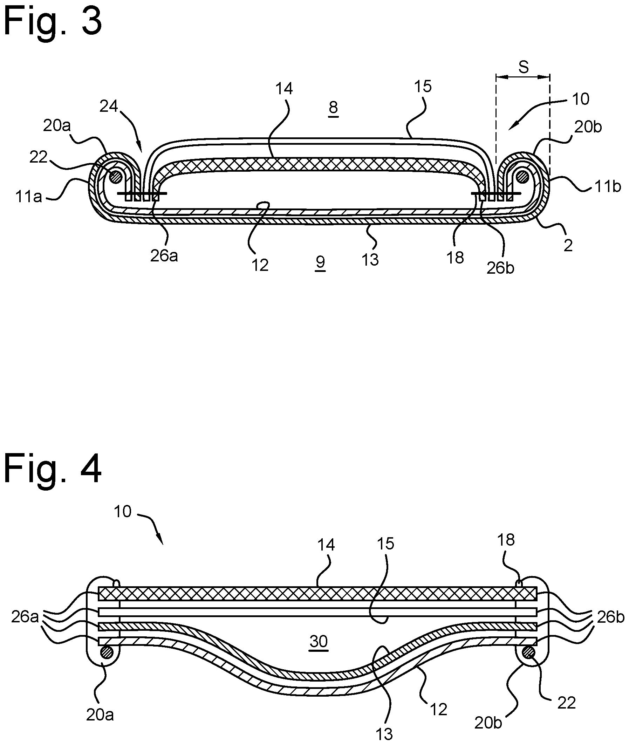

Fig. 3 shows a cross-section through the gusset 10 at the rear crotch region 7b, taken in the direction III-III in Figure 2 showing the layers of the gusset 10. The body panel 2 forms an outermost garment layer 13 at the exterior side 9, facing away from the wearer. The top layer 15 forms an uppermost layer at the interior side 8 facing in a direction towards the wearer. Beneath the top layer 15 is an absorbent core layer 14 and a moisture barrier layer 12. The top layer 15, the absorbent core layer 14, the barrier layer 12 and the garment layer 13 are all connected together by stitches 18 to form lateral seams 20a, 20b. Leg elastics 22 are included within the lateral seams 20a, 20b.

In the illustrated example, the absorbent core layer 14 is co-extensive with the top layer 15 in the width direction. The barrier layer 12 and the garment layer 13 are also co-extensive with each other in the width direction and are wider than the absorbent core layer 14 and the top layer 15. As a result, the barrier layer 12 and the garment layer 13 wrap around the leg edges 11a, 11b of the gusset 10 and the joins 24 between the top layer 15 and the garment layer 13 face inwards at the interior side 8 at distance S from the leg edges 11a, 11b.

Fig. 4 shows a cross-section through the gusset 10 corresponding to Fig. 3 during a step of manufacture. The layers of the gusset 10 are stacked on top of each other with moisture barrier layer 12 at the bottom of the stack, followed by the garment layer 13 and the top layer 15, with the absorbent core layer 14 uppermost. The layers have side edges 26a, 26b, which are stitched together by stitches 18 to form the lateral seams 20a, 20b. The stitches 18 are overlock stitches, which lock around the side edges 26a, 26b and also capture leg elastics 22. A lumen 30 is formed between the top layer 15 and the garment layer 13.

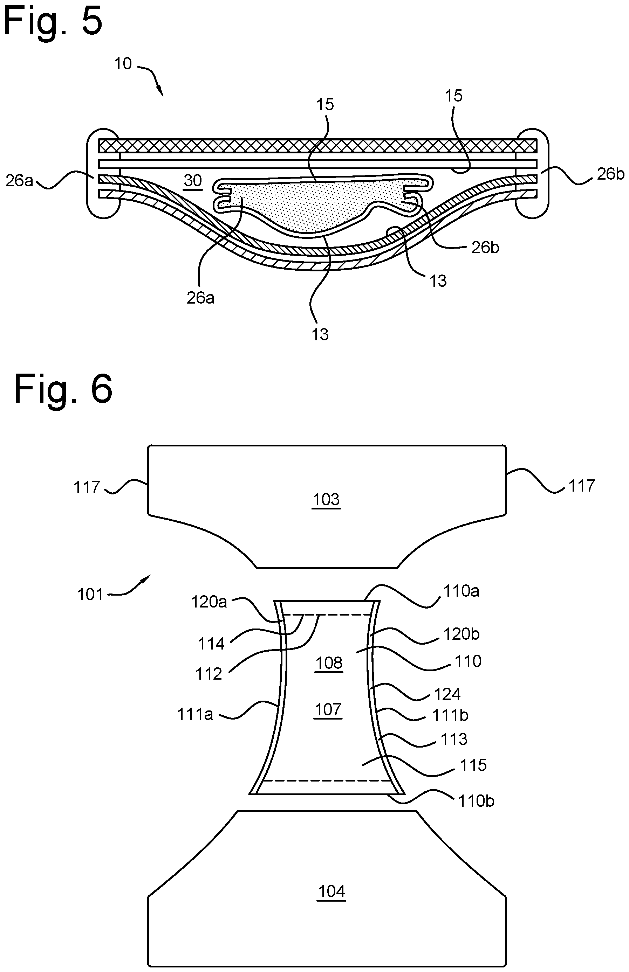

Once stitching of the gusset 10 is complete, the gusset is everted by passing the whole of the undergarment 1 through the lumen 30 between the garment layer 13 and the top layer 15. Fig. 5 shows the cross-section through the gusset 10 corresponding to Fig 4 as part of the undergarment 1 is being passed through the lumen 30. As can be seen, the top layer 15 and the garment layer 13 are outermost and once everted, the side edges 26a, 26b are at an interior of the gusset. It will be understood that the lumen 30 must be adequately large for the undergarment 1 to pass through.

The illustrated gusset 10 comprises just a top layer 15, absorbent core layer 14, moisture barrier layer 12 and garment layer 13. It will be understood that any number of additional layers may be provided depending upon requirements. Thus, further core layers may be provided with different sizes and absorbent characteristics. Wicking layers and distribution layers may also be provided. Any layers between the top layer 15 and the barrier layer 12 may be termed intermediate layers.

The top layer 15 comprises a water-permeable material thus allowing the body fluids to migrate to the underlying core layer 14. The top layer 15 may be constructed of any suitable fabric, including naturally derived fibres selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibres selected from the group consisting of polyamide, acrylic, polyester or elastane, such as a mixture of polyester and elastane. Further, the top layer 15 may be constructed of a blend or a mixture of naturally derived and/or synthetic fibres. The materials used for construction of the top layer should be soft and non-irritating to the skin and be readily penetrated by any body fluids. The top layer 15 is washable and may be of a textile material, such as a knitted material. The top layer 15 may have a basis weight of 80-300 gsm.

The absorbent core layer 14 comprises a washable knitted or woven material capable of absorbing fluid, such as woven or knitted microfibre or polymer, fabric formed from hydrophilic fibres, absorbent fibres. The absorbent core layer 14 may comprise natural or synthetic fibres as described above for the top layer 15 but may alternatively include polyester or polyamide. The core layer 14 may comprise between 20% and 100% natural or naturally derived fibres. The basis weight of the core layer 14 may be 180-600 gsm. In the illustrated example, the core laayer 14 is a tight knit interlock knitted fabric having a single-sided terry finish at the wearer facing interior side 8.

The moisture barrier layer 12 is washable and may comprise a film or laminate e.g. of extruded polymeric material or a woven or knitted material of any suitable construction to prevent liquid from migrating from the gusset 10 to the garment layer 13. The moisture barrier layer 12 in the illustrated variant, is a tightly woven activated carbon cloth that is sufficiently impermeable by nature. One suitable material is Flexzorb ^{®} from Chemviron SA. Laminates with polymer films may also be used. The activated carbon material may be highly desirable in reducing odours. The moisture barrier layer 12 may also comprise a coating of a moisture-impermeable material. The coating may be a polymer such as urethane wax or polyurethane provided for example on the surface facing away from the wearer of the moisture barrier layer 12.

The moisture barrier layer 12 may be breathable, to allow vapour to escape from the absorbent undergarment 1 while preventing liquid from passing through the fabric layer. Air and vapour permeability may be achieved by the woven or knitted material or by an additional rate controlling layer attached thereto.

Figure 6 shows an absorbent undergarment 101 in a disassembled state, viewed from the interior side 108 as in Figure 2. Like elements of Figures 1 to 5 are designated by the same reference numerals preceded by 100.

The undergarment 101 comprises a front panel 103, a rear panel 104 and a crotch panel 107. The crotch panel 107 has a front edge 110a, a rear edge 110b and leg edges 111a, 111b. Each leg edge 111a, 111b, thus connects the front edge 110a and the rear edge 110b. To form the undergarment 101, the front panel 103 is connected to the front edge 110a and the rear panel 104 is attached to the rear edge 110b. The front panel 103 is then connected to the rear panel 104 at side joints 117. All of the connections are made with conventional sewing techniques.

The crotch panel 107 comprises a gusset 110. The gusset 110 comprises an absorbent core layer 114 and a moisture barrier layer 112 (shown in broken lines) which terminate short of the front edge 110a and rear edge 110b of the crotch panel 107. The gusset 110 also comprises a garment layer 113 and a top layer 115, which do continue to the front edge 110a and rear edge 110b and are there connected to the front panel 103 and rear panel 104 respectively. The layers of the gusset 110 are all connected together with lateral seams 120a, 120b, whereby a join 124 is visible at the interior side 108, where the top layer 115 and the garment layer 113 meet.

The gusset 110 is otherwise identical in construction to the gusset of Figures 1 to 5. A distinction is that during fabrication, the gusset 110 may be easier to evert, since the front panel 103, 104 need not be present and do not thus need to be everted through the gusset lumen. Additionally, cutting out of the panels of the undergarment 101 may be more efficient in terms of material usage and waste.

The disclosure may be varied within the scope of the appended claims. For example, the materials and dimensions used for the different layers forming the absorbent insert may be varied, as indicated above.

## Claims

1. A washable absorbent undergarment comprising woven or knitted fabric, having a multi-layer gusset extending at least within a crotch region of the garment, the gusset comprising at least a body-facing top layer, a moisture barrier layer and a garment layer, with lateral seams joining side edges of the layers together, wherein the seams are formed in an everted state such that the side edges terminate at an interior of the gusset.

2. The garment of claim 1, wherein the seams are stitched seams, such as overlock stitched seams

3. The garment of claim 1 or claim 2, wherein at the seams, the garment layer is sandwiched between the barrier layer and the top layer.

4. The garment of any one of the preceding claims, wherein the barrier layer and garment layer are wider than the top layer, at least in the crotch region, and extend around the side edges of the gusset.

5. The garment of any one of the preceding claims, wherein joins between the garment layer and the top layer extend along a body facing surface of the garment.

6. The garment of any one of the preceding claims, wherein the gusset has a waisted shape, being narrowest at the crotch region and wider at a front edge and a rear edge.

7. The garment of any one of the preceding claims, wherein the garment layer extends beyond the gusset.

8. The garment of claim 7, wherein the garment layer comprises a single fabric panel extending throughout the undergarment.

9. The garment of any one of claims 1 to 7, wherein the garment layer is connected to further fabric panels to form the undergarment.

10. The garment of any one of the preceding claims, comprising leg elastics extending along the seams.

11. The garment of any one of the preceding claims, wherein the gusset further comprises an absorbent core layer, between the top layer and the moisture barrier layer.

12. The garment according to any one of the preceding claims, wherein a strip of hydrophobic material is located between the garment layer and the top layer at the lateral seam.

13. A multi-layer gusset, comprising woven or knitted fabrics, for a washable absorbent undergarment, the gusset comprising at least a body-facing top layer, a moisture barrier layer and a garment layer, with lateral seams joining side edges of the layers together, wherein the seams are formed in an everted state such that the side edges terminate at an interior of the gusset.

14. A method of manufacturing a multi-layer gusset, comprising woven or knitted fabric, for a washable absorbent undergarment, the method comprising:
a. providing a stack comprising in sequence, a moisture barrier layer, a garment layer, a body-facing top layer and an optional absorbent core layer;
b. joining side edges of the layers together to form lateral seams; and
c. everting the gusset such that the top layer and the garment layer are outermost.

15. The method of claim 14, further comprising attaching the garment layer to further fabric panels of garment material or to itself, to form an undergarment.
